# FASCICULE DE BREVET EUROPEEN

(11) **EP 0 714 910 B1**
(45) Date de publication et mention de la délivrance du brevet: **07.04.1999**
(21) Numéro de dépôt: 95402697.7
(22) Date de dépôt: 30.11.1995
(51) Int. Cl.: C07K 14/47, A61K 38/08

(54) **Utilisation d'un décapeptide à activité de type benzodiazépine pour la préparation de médicaments et de compléments alimentaires**
Verwendung eines Dekapeptids mit benzodiazepinischer Wirkung zur Herstellung von Arzneimitteln und Diätzusätzen
Use of a decapeptide with a benzodiazepine activity for the preparation of medicaments and dietary supplements

(30) Priorité: 30.11.1994 FR 9414362
(43) Date de publication de la demande: 05.06.1996
(73) Titulaire: Société Coopérative Agricole Laitière d'Artois et des Flandres, La Prospérité Fermière, 62000 Arras (FR)
(72) Inventeur: Miclo, Laurent, 54000 Nancy (FR); Perrin, Emanuel, F-54420 Saulxures-les-nancy (FR); Driou, Alain, F-54520 Laxou (FR); Boudier, Jean-Francois, F-62217 Agny (FR); Iung, Catherine, F-62000 Arras (FR); Linden, Guy, F-54180 Heillecourt (FR)
(74) Mandataire: Gillard, Marie-Louise

(56) Documents cités:
- JOURNAL OF FOOD SCIENCE., vol. 54, no. 5, Octobre 1989 CHICAGO US, pages 1225-1229, E MINAGAWA ET AL. 'Debittering mechanism in bitter peptides of enzymatic hydrolysates from milk casein by aminopeptidase T'
- BIOCHEMICAL AND BIOPHYSICAL RESEARCH COMMUNICATIONS., vol. 154, no. 3, 15 Août 1988 DULUTH, MINNESOTA US, pages 876-882, A AMETANI ET AL. 'Antibody response in bitter peptides of three different strains of mice to alpha-s1 casein analyzed by using proteolitic ynd sythetic peptides'
- BIOLOGICAL ABSTRACTS, vol. 93, no. 12, 1992 Philadelphia, PA, US; abstract no. 138117, A BECKER & G GRECKSCH 'd-Tyr-D-Phe3 (Pro-D-Phe-Pro-Gly) interacts specifically with amygdaloid-kindled seizures and is capable of preventing the learning deficit occurring after kindling' page AB-919;
- JOURNAL OF BIOLOGICAL CHEMISTRY, vol. 254, no. 7, 10 Avril 1979 MD US, pages 2446-2449, C ZIOUDROU ET AL. 'Opioid peptides derived from food proteins. The exorphins.'
- CHEMICAL ABSTRACTS, vol. 118, no. 19, 10 Mai 1993 Columbus, Ohio, US; abstract no. 190552g, S SEKIYA ET AL. 'Antihypertensive effect of tryptic hydrolysate of casein on normotensive and hypotensive volunteers ' page 840;

## Description

La présente invention est relative à l'utilisation d'un décapeptide de la caséine αₛ₁ pour la préparation de médicaments et de compléments alimentaires.

La caséine entière est un ensemble de protéines du lait qui a été largement étudié, par exemple par RIBADEAU-DUMAS (1). La caséine donne, par chromatographie sur DEAE-cellulose, les principales fractions qui sont dénommées respectivement : caséines γ, caséine κ, caséine β, caséine αₛ₁ et caséine αₛ₂. Les séquences en acides aminés de ces caséines sont bien connues ; en particulier celle de la caséine αₛ₁ a été déterminée par MERCIER *et al.* (2) et NAGAO *et al.* (3).

On sait que certains fragments peptidiques de caséine, notamment le décapeptide 91-100 de la caséine αₛ₁, sont amers et pourraient être responsables de l'amertume du fromage. Il a donc été proposé d'hydrolyser ces fragments peptidiques par l'aminopeptidase T pour diminuer, voire annihiler l'amertume des fragments peptidiques amers [Minagawa et al. (15)].

On sait également que certains fragments peptidiques de ces différentes caséines ont des activités biologiques diverses et notamment des activités opiacées ou antiopiacées. Ainsi, les peptides 90-96, 90-95, 91-96 et 91-95 de la caséine αₛ₁ ont une activité opiacée [ZIOUDROU *et al.* (4) et LOUKAS *et al.* (5)].et des hydrolysats trypsiques de caséine de lait, en tant qu'ingrédient dans une alimentation particulière, pourraient prévenir l'hypertension [Sekiya et al. (16)].

La demanderesse s'est intéressée à d'autres types d'activités de la caséine αₛ₁ et des fragments de celle-ci, en particulier à l'activité de type benzodiazépine.

Par "activité de type benzodiazépine" on entend notamment les propriétés anticonvulsivantes et anxiolytiques.

On a en effet trouvé par des tests *in vitro* sur le récepteur des benzodiazépines et par des tests comportementaux in *vivo* chez le rat que le décapeptide défini ci-après et l'hydrolysat trypsique total le contenant ont des propriétés anticonvulsivantes et anxiolytiques intéressantes.

Ainsi la présente invention a pour objet l'utilisation du décapeptide ayant la séquence en acides aminés : 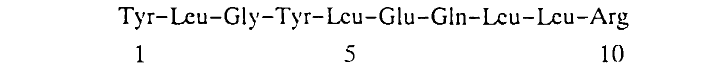 pour la préparation de médicaments à activité de type benzodiazépine, notamment pour le traitement des convulsions et de l'anxiété.

L'invention a également pour objet les compositions pharmaceutiques contenant à titre d'ingrédient actif une quantité efficace dudit décapeptide en combinaison avec un véhicule pharmaceutiquement acceptable.

L'invention concerne également les compléments alimentaires contenant à titre de principe actif ledit décapeptide ou l'hydrolysat trypsique total de la caséine αₛ₁ contenant ledit décapeptide ou une fraction de cet hydrolysat contenant ledit décapeptide. Ces compléments alimentaires conviennent pour supplémenter l'alimentation des personnes sujettes notamment aux convulsions ou à l'anxiété.

Le décapeptide ayant la séquence en acides aminés ci-après : 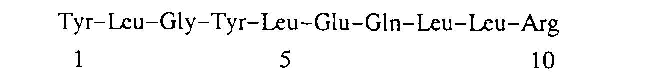 de masse moléculaire de 1 267 Da, correspond au peptide 91-100 de la caséine αₛ₁.

Il peut être obtenu à partir de la caséine αₛ₁ par hydrolyse enzymatique, en particulier à l'aide de la trypsine. Il peut être ensuite concentré ou isolé par chromatographie liquide haute performance (CLHP) en phase inverse, par chromatographie liquide haute performance d'échange d'anions ou par chromatographie de gel filtration de seuil 1800 Da ou par centrifugation sur membrane et autres techniques de séparation sur membrane (microfiltration, ultrafiltration, etc.)

Le décapeptide peut aussi être obtenu par synthèse peptidique selon les procédés bien connus de l'homme de l'art, tels que ceux décrits par exemple par MERRIFIELD (6).

La caséine entière est obtenue à partir du lait par précipitation acide et neutralisation à l'aide d'un alcali selon des procédés bien connus. Par exemple, on peut utiliser la méthode de NITSCHMANN *et al.* (7).

La caséine αₛ₁, utilisée comme produit de départ pour l'obtention de l'hydrolysat trypsique total et du décapeptide selon l'invention, peut être obtenue par les procédés classiques bien connus de l'homme du métier à partir du lait, de caséines entières, de caséinates et de concentrés de protéines totales du lait, obtenus par exemple selon les procédés décrits par THOMSON (8) et MAUBOIS (9).

Par exemple, on peut préparer la caséine αₛ₁ en mettant en oeuvre la méthode décrite par SANOGO *et al.* (10). Cette méthode est une méthode de fractionnement sur DEAE-cellulose utilisant un gradient discontinu de chlorure de calcium comme éluant. Elle présente l'avantage de fractionner rapidement l'ensemble des caséines. Elle peut être avantageusement mise en oeuvre avec, comme support échangeur d'anions, la DEAE-cellulose DE 52 [commercialisée par WHATMAN Ltd. Springficld, Grande-Bretagne] qui est une résine pré-gonflée ne nécessitant aucun pré-cycle acido-basique avant sa première utilisation.

En utilisant un autre type de résine DEAE-cellulose on peut éliminer en deux étapes seulement l'ensemble des caséines autres que la caséine αₛ₁ tout en augmentant la pureté et le rendement de la caséine αₛ₁ obtenue. Ainsi, on peut par exemple utiliser une résine sèche à la place d'une résine pré-gonflée, telle que par exemple la DEAE-cellulose DE 23 [commercialisée par WHATMAN cité ci-dessus]. En utilisant une résine sèche et en supprimant le cycle de prétraitement nécessaire pour obtenir une charge maximale, on limite l'efficacité de la charge de la résine et donc la fixation du substrat sur celle-ci.

Les médicaments utiles pour le traitement des convulsions ou de l'anxiété préparés avec le décapeptide défini selon l'invention peuvent être administrés par différentes voies d'administration, par exemple par voie orale ou par voie parentérale.

Pour une administration par voie orale, les compositions pharmaceutiques selon l'invention peuvent être sous forme de comprimés, gélules, poudres, granulés ou toute autre forme administrable par voie orale.

Les compositions pharmaceutiques selon l'invention peuvent en outre contenir des véhicules pharmaceutiques acceptables couramment utilisés pour la préparation de formes orales.

Pour l'administration par voie parentérale, on pourra utiliser des solutions salines isotoniques, des solutions injectables qui peuvent contenir des agents de dispersion compatibles.

L'hydrolysat trypsique total obtenu par action de la trypsine sur la caséine αₛ₁ dans les conditions indiquées précédemment comprend de 5 à 6 % en poids du peptide 91-100.

Il peut être utilisé comme principe actif soit dans des compléments alimentaires en combinaison avec des supports alimentaires de nature protéique ou glucidique, soit dans des produits alimentaires destinés à une alimentation particulière.

L'invention va être maintenant décrite plus en détail par les exemples ci-après non limitatifs.

### EXEMPLE 1 : Préparation du peptide 91-100 de la caséine αs1 (peptide CN αs1- (f91-100)x

### A - Préparation de la caséine αₛ₁

5 g de caséinate de sodium ont été dissous dans 100 ml de tampon acétate 20 mM, pH = 6,6 contenant de l'urée 3,3 M, de l'EDTA 35 mM et 0,1 % de 2-mercaptoéthanol puis on a ajouté 20 g de résine DEAE-cellulose DE 23 équilibrée dans 150 ml du même tampon et on a agité le mélange résultant pendant environ 15 min à 25° C. On a filtré sur büchner puis on a élué le rétentat avec 2 fois 250 ml du tampon acétate-urée-EDTA. Cette première filtration a permis d'éliminer une fraction F_{I} contenant les caséines γ, κ et β. On a ensuite ajouté 250 ml du tampon acétate/urée contenant 35 mM de CaCl₂ et on a répété l'opération ci-dessus ; on a ainsi pu éliminer une seconde fraction F_{II} qui contient la caséine αₛ₂. On a répété l'extraction ci-dessus en utilisant le tampon acétate/urée contenant 70 mM de CaCl₂ et on a isolé une fraction F_{III} qui contient la caséine αₛ₁.

A la fin de la procédure, la résine a été lavée avec le tampon initial et conservée à +4°C dans cette solution renfermant 0,2% d'azide de sodium. Ainsi stockée, la résine peut être de nouveau utilisée directement, sans régénération, pour une autre extraction.

Les filtrats F_{I}, F_{II}, F_{III}, dialysés contre de l'eau ultra-pure puis lyophilisés, ont été soumis à une électrophorèse en gel de polyacrylamide-urée afin de visualiser le fractionnement.

En absence de CaCl₂, les caséines γ, κ et β ont été élués (F_{I}). Quant à la caséine αₛ₂, elle est décrochée par une concentration en CaCl₂ de 35 mM (F_{II}). La troisième fraction (F_{III}), éluée à une concentration en CaCl₂ égale à 70 mM, contient les caséines αₛ₁ et αₛ₀. Cette dernière n'est qu'une forme mineure de la caséine αₛ₁ différant de la forme majeure par la présence d'un groupement phosphate supplémentaire en position 41.

Le degré de pureté de la caséine αₛ₁ ainsi obtenue est supérieur à 96 %. Elle a été utilisée telle que dans la suite du procédé sans étape de purification supplémentaire.

### B- Préparation de l'hydrolysat trypsique de la caséine αₛ₁

L'hydrolyse trypsique de la caséine αₛ₁ à la concentration de 0,2% (p/v) dans 25 ml de tampon NH₃/HCOOH (57 mM/43 mM) pH = 8,5 à 37°C a été effectuée avec, comme enzyme, la trypsine pancréatique bovine immobilisée sur billes d'agarose et traitée à la N-tosyl L-Phe chlorométhylcétone (TPCK) (80 unités/ml de billes). On a choisi une concentration enzymatique égale à 8,5 unités Na-benzoyl-L-arginine éthyl ester et un temps d'hydrolyse égal à une heure suivi d'une centrifugation à +4°C pendant 5 min à 1800 g.

Deux aliquotes enzymatiques ont été prélevés afin de réaliser deux hydrolyses en parallèle. Les sumageants provenant des hydrolyses, centrifugés pour éliminer l'enzyme insoluble, ont été réunis. Neuf évaporations entrecoupées de lavages à l'eau ont été réalisées. Après la dernière évaporation, l'hydrolysat, débarrassé ainsi de la majeure partie de ses sels, a été repris dans un minimum d'eau, lyophilisé et stocké à -30°C.

### C- Fractionnement en CLHP-phase inverse en gradient d'acétonitrile et sous conditions isocratiques

L'hydrolysat de caséine obtenu à l'étape B a été soumis à deux étapes de purification.

### C₁ - purification sur colonne C₁₈

La première étape de purification est un fractionnement de l'hydrolysat trypsique de caséine αₛ₁ en CLHP-phase inverse sur colonne C₁₈ (Merck, Darmstadt, Allemagne) 250 x 4 mm, 100 Å, 5 µm, en utilisant un gradient d'acétonitrile dans l'eau (en présence de 0,1% d'acide trifluoroacétique ou TFA) allant de 5% d'acétonitrile à 40% sur 70 minutes.

Le pic s'éluant vers 61 min (environ 32% v/v d'acétonitrile) et contenant entre autres le peptide CNαₛ₁-(f91-100) a été collecté. L'acétonitrile et le TFA sont évaporés sous pression réduite avant lyophilisation.

### C₂- purification sur colonne C₄

Le pic précédemment collecté est ensuite purifié par CLHP-phase inverse sur une colonne Delta Pak C₄ 150 x 3,9 mm, 300 Å, 5 µm (Waters, Midford, Royaume-Uni) en conditions isocratiques d'acétonitrile 25% v/v dans l'eau en présence de 0,1% de TFA. Le peptide CNαₛ₁-(f91-100) s'élue à environ 10 min. Il est collecté et lyophilisé. Le lyophilisat est repris dans l'eau et de nouveau lyophilisé.

La nature du peptide est confirmée par spectrométrie de masse F.A.B. ("fast atom bombardment" ou bombardement électronique) (masse moléculaire déterminée : 1267,1 Da) et par sa composition en acides aminés déterminée par la méthode à la ninhydrine de HAMILTON *et al.* (11).

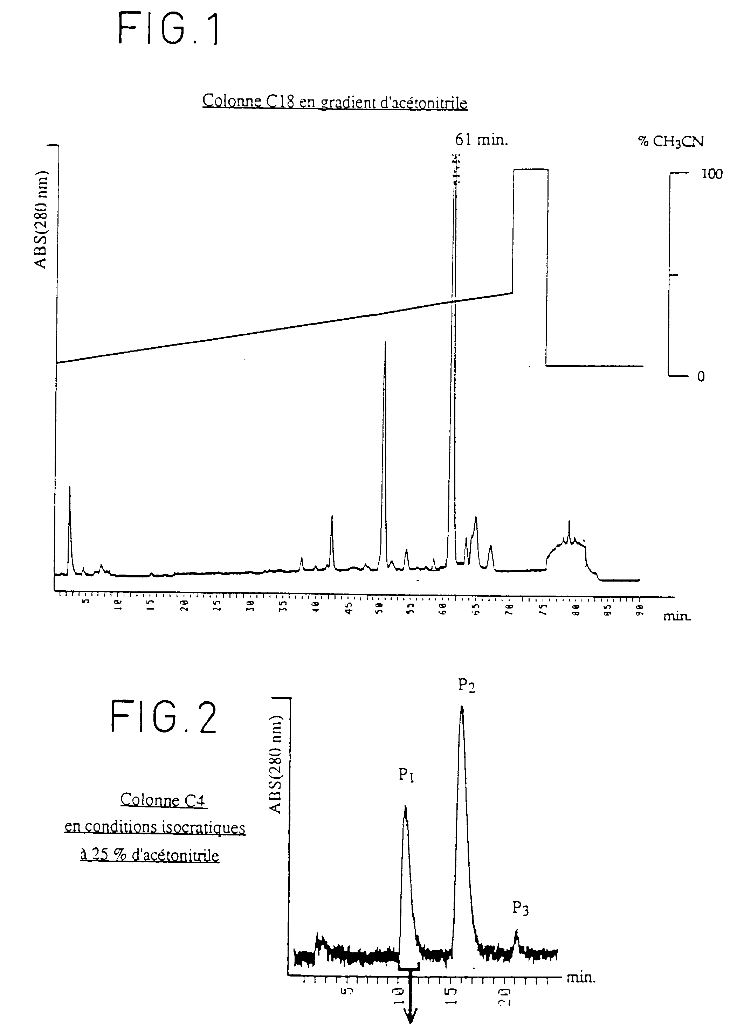

Les profils d'élution sur colonnes C₁₈ et C₄ sont représentés respectivement sur les figures 1 et 2 sur lesquelles l'absorbance à 280 nm est portée en ordonnées et le temps d'élution en abscisses.

### EXEMPLE 2 : Obtention de fractions enrichies en peptide CNαₛ₁ (f91-100) par différentes voies

### A- Fractionnement de l'hydrolysat trypsique de caséine αₛ₁ sur membrane par centrifugation

En vue d'une pré-purification rapide de l'hydrolysat trypsique de caséine, on a réalisé un fractionnement grossier par centrifugation sur membrane ayant un seuil de coupure moyen de 2000 Da (AMICON YM1).

2 mg du produit obtenu à l'exemple 1 (étape B) dissous dans 1 ml d'eau ultra-pure sont placés dans un système de micropartition Amicon de type MPS-1. Le temps de centrifugation est de 40 ou 60 min pour un champ d'accélération de 3900 g.

Après écoulement du temps de centrifugation, les deux systèmes de micropartition sont retirés de la centrifugeuse et les perméats et rétentats sont stockés dans des micro-tubes. Le contenu des différents micro-tubes est ensuite lyophilisé.

Le peptide CN S1-(f91-100) de faible masse moléculaire est passé à travers la membrane et se retrouve donc dans le perméat.

### B- Fractionnement de l'hydrolysat trypsique de caséine αₛ₁ par chromatographie liquide haute performance d'échange d'anions (MONO Q, HR 5/5)

L'utilisation d'une chaîne de chromatographie de FPLC (Pharmacia Uppsala, Suède) permet une séparation beaucoup plus fine des peptides mais seulement à un niveau semipréparatif (injection de 1 à 2 mg d'hydrolysat trypsique de caséine αₛ₁).

L'hydrolysat obtenu à l'exemple 1 (étape B)) a été fractionné par un gradient de 0 à 30% de B dans A pendant 45 min. puis de 30% à 100% de B dans A pendant 30 min. (A: Tris-HCI 20 mM pH 8,0; B: Tris-HCl 20 mM pH 8,0 avec 350 mM de NaCl). Le peptide CN αₛ₁ -(f91-100) de faible masse moléculaire présentant un nombre faible d'acides aminés chargés a été élué au niveau de la fraction correspondant au volume mort.

### C - Fractionnement de l'hydrolysat trypsique de caséine αₛ₁ par chromatographie de gel filtration de seuil 1800 Da

Cinq à dix milligrammes d'hydrolysat ont été déposés sur un lit de gel de Biogel P2 (Pharmacia, Uppsala, Suède) (diamètre 1 cm, hauteur 40 cm). L'élution a été réalisée sous pression atmosphérique par de l'eau ultra-pure à un débit de 5 à 10 ml/h.

Deux fractions (F1 et F2) ont ainsi été obtenues. La fraction F1 contenait les peptides exclus du gel (masse moléculaire supérieure à 1800 Da) et la fraction F2 contenant les peptides filtrés par le gel (dont le peptide CNαₛ₁-(f91-100)).

### TESTS PHARMACOLOGIQUES

### A - TESTS IN VITRO SUR LE RECEPTEUR DES BENZODIAZEPINES

L'expérience a été réalisée à l'aide d'un coffret commercialisé par Dupont de Nemours (NENQUEST TM, Drug Discovery System, NED-002). La méthode est basée sur la compétition entre un ligand radioactif du récepteur central des benzodiazépines et une molécule à tester. Si la molécule a une affinité pour le récepteur, elle déplace le ligand marqué fixé au récepteur. Ce déplacement du ligand en fonction de la concentration en molécule à tester ajoutée permet de déterminer l'IC₅₀ de cette molécule c'est-à-dire la concentration pharmacologique qui permet d'obtenir 50% de l'effet maximum.

Le ligand utilisé est le ³H-méthyl-flunitrazépam, une benzodiazépine qui possède une forte spécificité pour le récepteur (Ki=1,2 nM) et une faible fixation aspécifique. On a incubé des préparations membranaires commerciales des récepteurs centraux des benzodiazépines avec des concentrations croissantes de l'échantillon à tester et une concentration fixe en radioligand. L'incubation dure une heure à +4°C. Cette température est volontairement choisie basse afin de limiter l'association/dissociation entre le récepteur et le radioligand. Le flunitrazépam a un temps de demi-association de 834 s à 0°C et de 12 s à 35°C (SPETH *et al.,* (12). Après une heure, le mélange a été passé sur filtre Whatman GF/B. Ces filtres retiennent les membranes et les ligands (benzodiazépines, fragment(s) peptidique(s) de l'hydrolysat trypsique de caséine αₛ₁) qui y sont fixés. Après lavage, les filtres sont mis dans des flacons de comptage contenant du liquide à scintillation. L'émission de rayonnement β est comptée par un compteur à scintillation liquide.

Si le ligand tritié est déplacé par la molécule testée, on observe une baisse du taux de radioactivité présente sur le filtre.

Afin d'éliminer la radioactivité due à une liaison aspécifique entre le ligand tritié et le récepteur, un essai a été réalisé en incubant les membranes, le ligand tritié à la même concentration que celle utilisée pour les essais où est testée une molécule dont on veut déterminer l'IC₅₀ et du flunitrazépam non marqué à une concentration 500 fois en excès. La radioactivité résiduelle n'est donc due qu'à la fixation aspécifique puisqu'on suppose que tous les sites sont occupés par le flunitrazépam non marqué.

On peut définir une valeur B égale au pourcentage de radioactivité fixée sur les récepteurs pour chaque concentration de molécule à tester par rapport à un essai réalisé avec seulement le flunitrazépam marqué et les membranes (maximum de radioactivité). En portant le logarithme décimal de B/100-B en fonction du logarithme décimal de la concentration en molécule à tester ajoutée, on peut déterminer l'IC₅₀ de cette molécule.

Plus une IC₅₀ est basse plus l'affinité de la molécule pour le récepteur est élevée. Pour l'hydrolysat trypsique total de caséine αₛ₁ (non purifié), la meilleure IC₅₀ vis-à-vis du récepteur central des benzodiazépines mise en évidence à ce jour est de 78 µM.

Pour le peptide CNαₛ₁ -(f91-100) purifié selon la méthode décrite dans l'exemple 1 étape C, l'IC₅₀ est d'environ 88 µM.

Ces tests ont été également réalisés avec le décapeptide obtenu par synthèse peptidique selon MERRIFIELD (6). On a trouvé que l'IC₅₀ du peptide de synthèse est de 370 µM contre 88 µM pour le décapeptide d'origine naturelle.

Pour les fractions enrichies en peptide CN S1-(f91-100) selon les méthodes décrites dans l'exemple 2, l'activité a également été retrouvée.

### B - TESTS IN VIVO CHEZ LE RAT WISTAR

### B₁ Activité anticonvulsivante

Le pentylènetétrazole est une molécule agissant au niveau des canaux à chlorure du récepteur GABA A. En obstruant ce type de canal, il provoque des symptômes ressemblant à ceux de la crise d'épilepsie. Les molécules de type anti-épileptiques s'opposent à cet effet en réduisant les conséquences dues à la présence du pentylènetétrazole.

Ces tests ont été réalisés dans la salle d'élevage pendant la phase d'obscurité. La quantité d'hydrolysat trypsique de caséine αₛ₁ (D₁) injectée a été de 3 mg/ kg de rat. La voie d'injection utilisée était la voie intrapéritonéale. L'hydrolysat trypsique total a été mis en solution dans du diméthyl isosorbide éther à 25% dans l'eau; ce mélange présente l'avantage d'être amphiphile. Le diméthyl isosorbide, miscible à l'eau, présente l'avantage de solubiliser des molécules très hydrophobes.

Trois paramètres ont été étudiés afin de décrire l'importance de la crise :
- La sévérité de la crise qui s'évalue d'après l'échelle de RACINE (13).
   Stade 0: aucune réponse comportementale visible.
   Stade 1: immobilité accrue de l'animal.
   Stade 2: balancement de la tête et/ou mouvements cloniques de la mâchoire.
   Stade 3: stade 2 + redressement sur les pattes postérieures.
   Stade 4: stade 3 + mouvements cloniques des pattes antérieures.
   Stade 5: crise complète. Redressement sur les pattes postérieures, clonus des pattes antérieures et de la face, perte de l'équilibre.
- La latence qui est le temps en secondes séparant l'injection du pentylènetétrazole et l'apparition du premier signe de crise.
- La durée est le temps en secondes séparant le premier du dernier signe de crise.

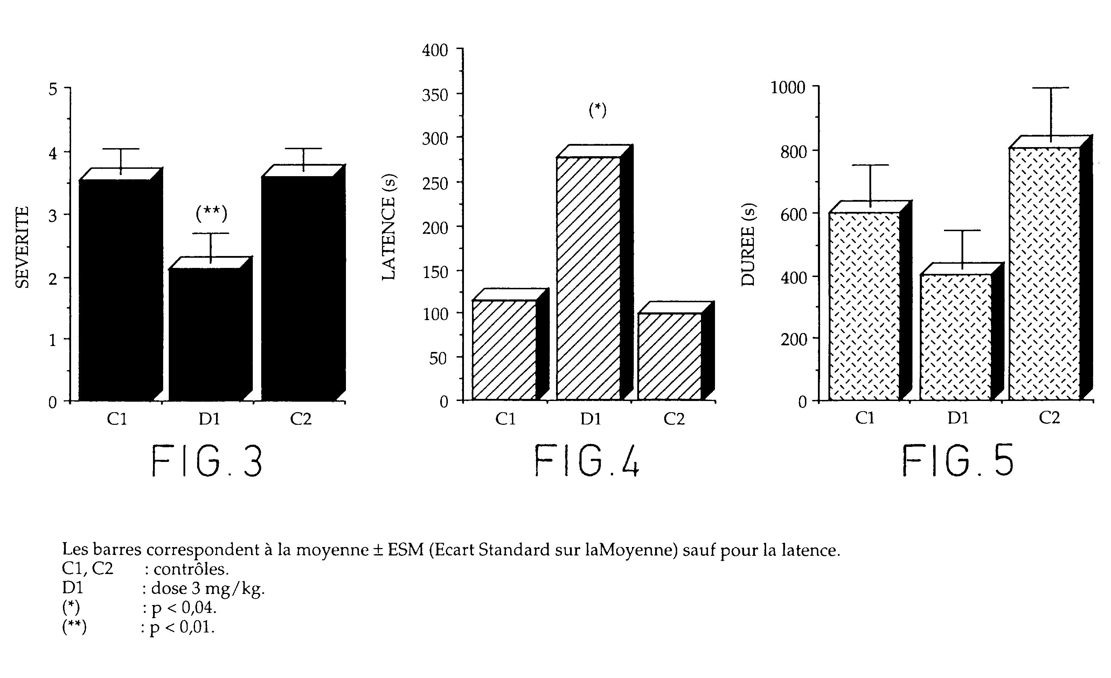

Les résultats obtenus avec l'hydrolysat trypsique total figurent sur les figures 3, 4 et 5 sur lesquelles on a indiqué respectivement :
la sévérité (figure 3)
la latence (figure 4)
la durée (figure 5)

Au niveau de la sévérité, les contrôles (C₁) et (C₂) effectués montrent une relative constance, ce qui valide les observations réalisées avec l'hydrolysat. Avec la dose (3 mg/kg) d'hydrolysat trypsique injecté, on a noté une diminution accrue de la sévérité de la crise. Les moyennes passent de 3,53 ± 0,40 à 2,13 ± 0,47. L'analyse statistique au moyen du test de WILCOXON réalisée en prenant comme référence le contrôle antérieur au test de l'hydrolysat donne, avec un Z=2,69, une tendance significative (p < 0,01) à la réduction de la crise (réduction de 40%). Le contrôle postérieur au test de l'hydrolysat, en revenant au niveau du contrôle précédent montre que la diminution observée de la sévérité avec l'hydrolysat n'est pas due à un phénomène de répétition ou d'accoutumance.

En ce qui concerne la latence, pour la dose 3 mg/kg, les médianes passent de 114 s, pour le contrôle antérieur, à 277 s. Avec un Z = 2,17 (p < 0,04) ceci correspond à une tendance significative à l'augmentation du temps séparant l'injection du pentylènetétrazole du premier signe comportemental de crise.

Pour la durée, l'injection d'hydrolysat trypsique de caséine αₛ₁ à 3 mg/kg semble aller dans le sens d'une réduction de la durée de crise; néanmoins cette réduction (602 ± 130 s. à 404 ± 123 s.) n'est pas significative (Z = 1,10). La substance injectée a un effet protecteur, puisque le pentylènetétrazole provoque des crises moins fortes, retardées et dont la durée semble baisser. L'effet est analogue à celui des substances qui potentialisent l'action GABAergique comme les benzodiazépines.

### B₂ Activité anxiolytique

Pour mesurer l'anxiété chez le rat, on a utilisé le test du labyrinthe en croix surélevé décrit par PELLOW *et al.,* (14) basé sur la néophobie du rat. Le labyrinthe en croix comporte quatre branches de 50 cm de long et 10 cm de large. Les deux bras fermés sont entourés de murs de 25 cm de haut (le dessus restant ouvert pour l'observation) et l'ensemble est à 50 cm du sol. Le centre du labyrinthe communique avec les deux bras ouverts et les deux bras fermés. En lumière tamisée, moins un rat est anxieux, plus il aura tendance à explorer les bras ouverts ce qui ne correspond pas à son comportement naturel.

Le nombre d'entrées effectuées par l'animal dans les bras ouverts, les bras fermés ainsi que le nombre d'entrées total sont des paramètres importants. Le temps passé dans chaque type de branches est également comptabilisé. D'autres paramètres (22 au total) tels que le nombre de redressements, le nombre de toilettage (signes d'anxiété) ou la latence de la première entrée dans une branche ouverte ou fermée sont pris en compte. L'observation de l'animal après qu'il fut placé au centre du dispositif dure 5 min. L'ensemble de son comportement est filmé par caméra vidéo, procédure qui permet d'éviter les erreurs.

On a observé 20 rats représentant le lot témoin, 20 rats recevant du diazépam (DZP) à 2 mg/kg comme contrôle positif et 20 rats traités avec l'hydrolysat trypsique de caséine αₛ₁ (HT) à 3 mg/kg (dose déterminée par rapport à l'expérience des convulsions).

Le lot témoin (CTR) a reçu une injection intrapéritonéale de solvant seul, celui-ci est un mélange de gélatine et de mannitol (0,5%/5%) dissous dans de l'eau. Il permet de mettre en suspension le diazépam qui est insoluble dans les solvants classiquement injectables (eau, NaCl 9 %o, éthanol 10 %). L'injection chez les animaux de tous les lots s'effectue une demi-heure avant le passage dans le labyrinthe en croix. Pour démarrer l'expérience, le rat est placé au centre de l'appareillage.

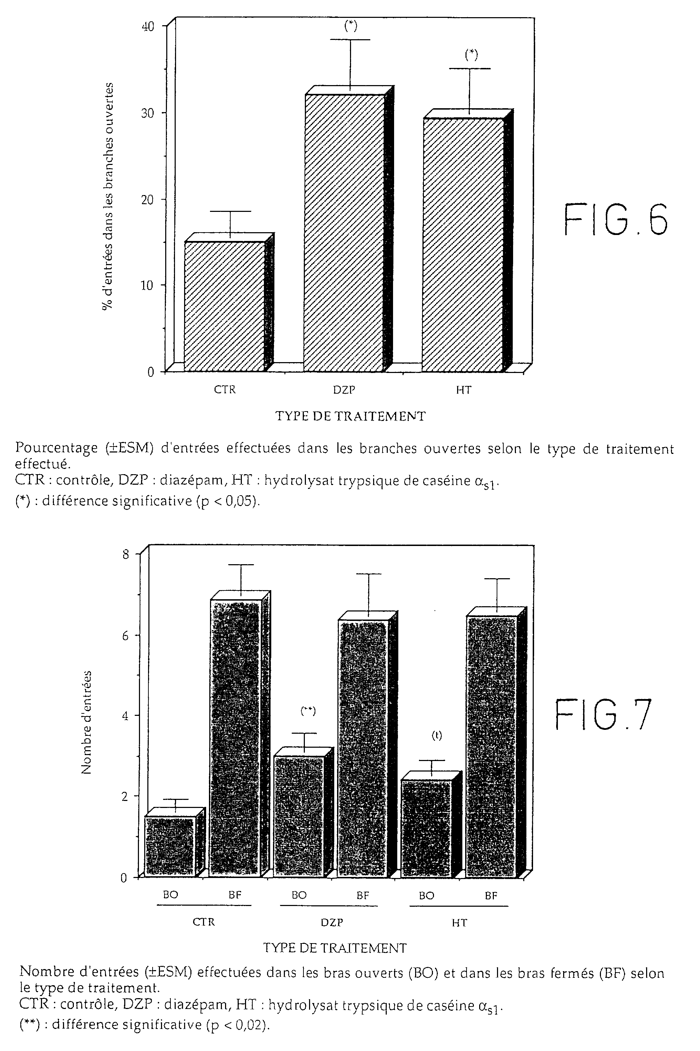

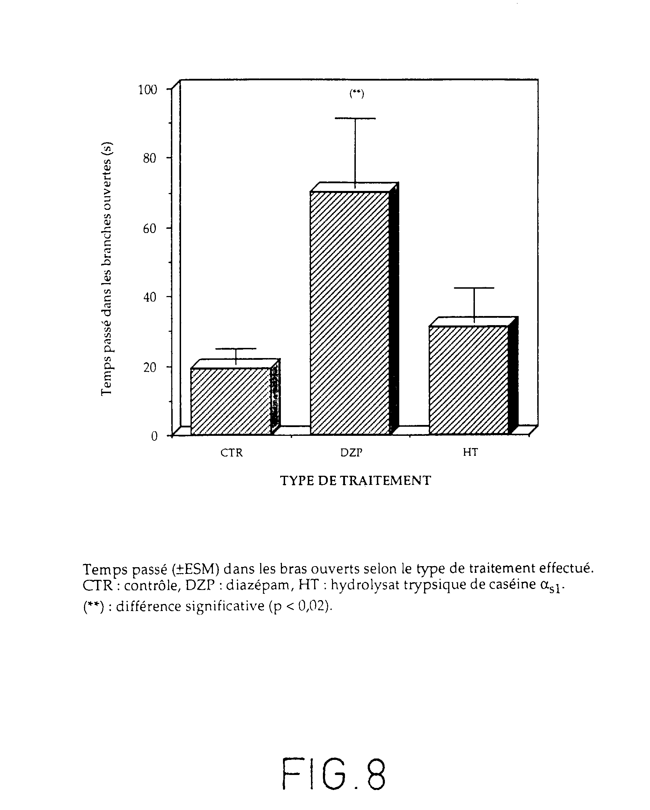

Les résultats obtenus sont reportés sur les figures 6, 7 et 8 sur lesquelles on a indiqué en fonction du type de traitement:
- le pourcentage d'entrées dans les branches ouvertes (figure 6) ;
- le nombre d'entrées effectuées dans les bras ouverts (BO) et les bras fermés (BF) (figure 7)
- le temps passé dans les branches ouvertes (figure 8).

Les rats traités au diazépam (DZP) effectuent 32,1±5,9% de leurs entrées dans les branches ouvertes alors que les contrôles n'en effectuent que 15,1±3,0% (p<0,05). Pour les rats traités par l'hydrolysat (HT), 29,3±5,3% de leurs entrées se font dans les branches ouvertes (p<0,05). Il faut noter que c'est le nombre d'entrées dans les branches ouvertes qui augmente pour le diazépam (3,0±0,4) et l'hydrolysat (2,4±0,4) (contrôle: 1,5±0,3) alors que le nombre d'entrées dans les branches fermées ne varie pas significativement.

Les rats traités au diazépam passent plus de temps dans les branches ouvertes (70,1± 19,9 s; p<0,02) que les contrôles (19,3±4,4 s). Pour les rats traités par l'hydrolysat (31,1±10,1 s), l'augmentation par rapport au lot contrôle n'est pas statistiquement significative.

On peut donc conclure que dans ce test, en n'étudiant que les paramètres majeurs, l'hydrolysat trypsique de caséine αₛ₁ a une action comparable à celle du diazépam mais avec un effet moins important; ceci n'est pas surprenant puisqu'il s'agit d'un hydrolysat brut.

### Références bibliographiques

**(1)** RIBADEAU-DUMAS, B., 1991, Physicochimie et biochimie des protéines du lait. Données récentes, Lait, 71, 133-139.
**(2)** MERCIER, J.C., GROSCLAUDE, F., and RIBADEAU-DUMAS, B., 1971, Structure primaire de la caséine αₛ₁ bovine. Séquence complète, Eur. J. Biochem., 23, 41-51.
**(3)** NAGAO, M., MAKI, M. SASAKI, R., and CHIBA, H., 1984, Isolation and sequence analysis of bovine αₛ₁-casein cDNA clone, Agric. Biol. Chem., 48, 1663-1667.
**(4)** ZIOUDROU, C., STREATY, R.A., and KLEE, W.A., 1979, Opioid peptides derived from food proteins : the exorphins, J. Biol. Chem., 254, 2446-2449.
**(5)** LOUKAS, S., VAROUCHA, D., ZIOUDROU, C., STREATY, R.A., and KLEE, W.A., 1983, Opiod activities and structure of α-casein-derived exorphins, Biochemistry, 22, 4567-4573.
**(6)** MERRIFIELD, R.B., 1963, Solid phase peptide synthesis I. Synthesis of a tetrapeptide, J. Amer. Chem. Soc., 85, 2149-2154.
**(7)** NITSCHMANN, H.S., et LEHMANN, W., 1947 Zum Problem der Labwirkung auf Casein, Helv. Chim. Acta, 130, 804.
**(8)** THOMSON, A.R, 1984, Recent developments in protein recovery and purification, J. Chem. Tech. Biotechnol., 34B, 190-198.
**(9)** MAUBOIS, J.L., 1984, Separation extraction and fractionation of milk protein components, Lait, 64, 485-495.
**(10)** SANOGO, T., PAQUET, D., AUBERT, F., and LINDEN, G., 1989, Purification of αₛ₁-casein by Fast Protein Liquid Chromatography, J. Dairy Sci., 72, 2242-2246.
**(11)** HAMILTON, P.B., 1963, Ion exchange chromatography of amino acids. Asingle column, high resolving, fully automatic procedure, Anal. Chem., 35, 2055-2063.
**(12)** SPETH, R.C., WASTEK, G.J., and YAMAMURA, H.I., 1978, Benzodiazepincs binding in human brain characterization using ³H flunitrazepam. Life Sci., 22, 859-866.
**(13)** RACINE, R.J., 1972, Modification of seizure activity by electrical stimulation. II. Motor seizure, Electroenceph. Clin. Neurophysiol., 32, 281-294.
**(14)** PELLOW, S., CHOPIN, P., FILE, S.E., and BRILEY, M., 1985, Validation of open : closed arm entries in an elevated plus-maze as a measure of anxiety in the rat. J. Neurosci. Mcthods, 14, 149-167.
**(15)** MINAGAWA, E., KAMINOGAWA, S., TSUKASAKI, F., and YAMAUCHI, K., 1989, Dcbittering Mechanism in Bitter Peptides of Enzymatic Hydrolysates from Milk Cascin by Aminopeptidase T, J. of Food Science, 54, (5), 1225-1229.
**(16)** SEKIYA, S. and al., 1993, Chemical Abstract, 118 : 190552 g.

## Revendications

1. Utilisation du décapeptide avant la séquence en acides aminés ci-après : 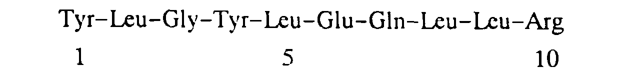 pour la préparation de médicaments à activité de type benzodiazépine, utiles notamment pour le traitement des convulsions et de l'anxiété.

2. Compositions pharmaceutiques, caractérisées en ce qu'elles contiennent à titre de principe actif une quantité efficace du décapeptide ayant la séquence en acides aminés ci-après : 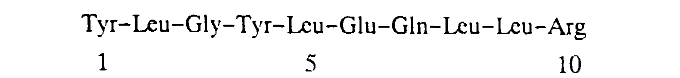 en combinaison avec un véhicule pharmaceutiquement acceptable.

3. Compléments alimentaires utiles pour supplémenter l'alimentation des personnes sujettes notamment aux convulsions et à l'anxiété, caractérisés en ce qu'ils contiennent une quantité efficace du décapeptide ayant la séquence en acides aminés ci-après : 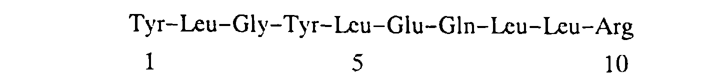 ou d'un hydrolysat trypsique total de caséine αₛ₁ le contenant ou d'une fraction de cet hydrolysat le contenant en combinaison avec des supports alimentaires de nature protéique ou glucidique.

4. Produits alimentaires destinés à l'alimentation des personnes sujettes notamment aux convulsions et à l'anxiété contenant une quantité efficace du décapeptide ayant la structure en acides aminés ci-après : 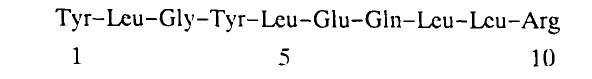 ou d'un hydrolysat trypsique total de caséine αₛ₁ le contenant ou d'une fraction de cet hydrolysat le contenant.

## Patentansprüche

1. Verwendung des Decapeptids mit der folgenden Aminosäuresequenz 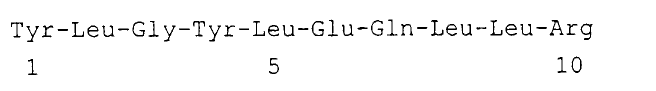 für die Herstellung von Arzneimitteln vom Wirkungstyp der Benzodiazepine, die insbesondere für die Behandlung von Krämpfen und Angst geeignet sind.

2. Pharmazeutische Zusammensetzungen, dadurch gekennzeichnet, daß sie als Wirkstoff eine wirksame Menge des Decapeptids mit der folgenden Aminosäuresequenz 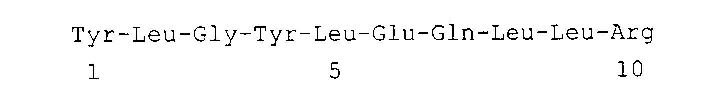 in Kombination mit einem pharmazeutisch akzeptablen Vehikel enthalten.

3. Lebensmittelergänzungen, die für die Ergänzung der Ernährung von Personen geeignet sind, die zu Krämpfen und Angst neigen, dadurch gekennzeichnet, daß sie eine wirksame Menge des Decapeptids mit der folgenden Aminosäuresequenz 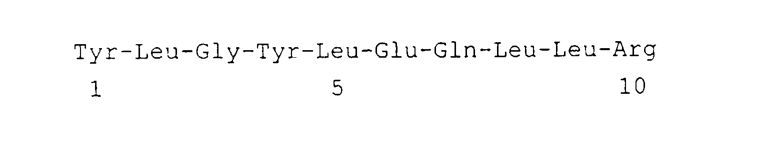 oder ein vollständiges Trypsin-Hydrolysat von αₛ₁-Ca-sein, das dieses Decapeptid enthält, oder eine Fraktion dieses Hydrolysats, die dieses Decapeptid enthält, in Kombination mit Lebensmittelträgern in Proteinform oder Kohlenhydratform enthalten.

4. Lebensmittelprodukte, die für die Ernährung von Menschen vorgesehen sind, die zu Krämpfen und Angst neigen, die ein wirksame Menge des Decapeptids mit der folgenden Aminosäurestruktur 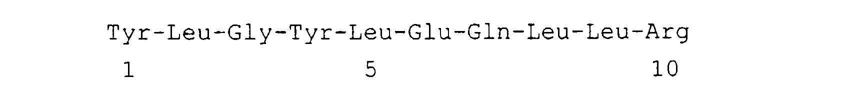 oder eines vollständigen Trypsin-Hydrolysat von αₛ₁-Ca-sein, das dieses Decapeptid enthält, oder eine Fraktion dieses Hydrolysats, die dieses Decapeptid enthält, enthalten.

## Claims

1. The use of the decapeptide having the following sequence of amino acids: 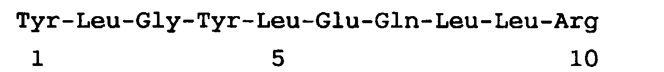 in the preparation of medication having benzodiazepine type activity, useful in particular for the treatment of convulsions and of anxiety.

2. Pharmaceutical compositions, characterized in that they contain as active ingredient an effective quantity of the decapeptide having the following sequence of amino acids: 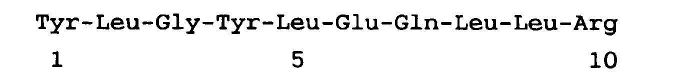 in combination with a pharmaceutically acceptable vehicle.

3. Food supplements, useful for supplementing the alimentation of people subject in particular to convulsions and to anxiety, characterized in that they contain an effective quantity of the decapeptide having the following sequence of amino acids: 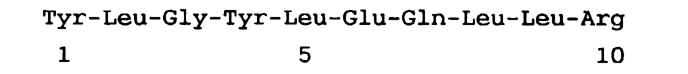 or a total trypsin hydrolysate of αs1 casein containing it or a fraction of said hydrolysate containing it in combination with protein or sugar type food media.

4. Foodstuffs for the alimentation of people subject in particular to convulsions and to anxiety containing an effective quantity of the decapeptide having the following structure of amino acids: 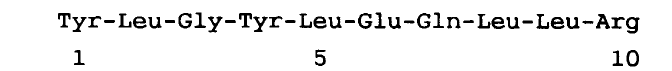 or a total trypsin hydrolysate of αs1 casein containing it or a fraction of said hydrolysate containing it.
